# EUROPÄISCHE PATENTSCHRIFT

(11) **EP 0 408 955 B1**
(45) Veröffentlichungstag und Bekanntmachung des Hinweises auf die Patenterteilung: **09.03.1994**
(21) Anmeldenummer: 90112512.0
(22) Anmeldetag: 30.06.1990
(51) Int. Cl.: A61M 1/30, A61M 1/10

(54) **Verfahren und Vorrichtung zur Steuerung von Blutpumpen bei einem extrakorporalen Kreislauf mit einer Single-Needle-Anordnung**
Procedure and device for controlling a blood pump of an extracorporal circuit with a single needle system
Procédé et dispositif pour contrôler une pompe à sang d'un circuit extracorporel muni d'un système single needle

(30) Priorität: 19.07.1989 DE 3923836
(43) Veröffentlichungstag der Anmeldung: 23.01.1991
(73) Patentinhaber: Fresenius AG, 61350 Bad Homburg (DE)
(72) Erfinder: Polaschegg, Hans-Dietrich, Dr., D-6370 Oberursel (DE)
(74) Vertreter: Fuchs Mehler Weiss

(56) Entgegenhaltungen:
- EP-A- 0 104 895
- EP-A- 0 229 271
- MEDICAL & BIOLOGICAL ENGINEERING & COMPUTING Band 17, Nr. 5, September 1979, Seiten 578-582, GB; J.H.MEIJER et al.: "Analysis of recirculation in single-needle heamodialysis" Seite 581, linke Spalte, Absatz 2; Seite 582, rechte Spalte, Absatz 1

## Beschreibung

Die Erfindung bezieht sich auf ein Verfahren und eine Vorrichtung zur Steuerung von Blutpumpen bei einem extrakorporalen Kreislauf mit einer Single-Needle-Anordnung. Im speziellen betrifft die Erfindung extrakorporale Dialysekreisläufe.

Die Verwendung einer Single-Needle-Anordnung oder einer Anordnung mit einer Einzelnadel bei der Dialyse ist seit langer Zeit bekannt. Bei den bisherigen Entwicklungen wurde stets versucht, die Funktionsfähigkeit der Gesamtanlage zu verbessern und vershciedene unterschiedliche Probleme zu beseitigen, so wurde beispielsweise die jeweilige Steuerung der Pumpen zur Durchführung der Arbeitstakte verbessert. Ein grundsätzlich bei derartigen Single-Needle-Anordnungen auftretendes Problem besteht bei allen bisher bekannten Anordnungen und wurde bezüglich einer Lösung bisher etwas zurückgestellt, da andere Schwierigkeiten und Nachteile vorrangig erschienen. Dieses Problem betrifft die Rezirkulation bei der Durchführung von Single-Needle-Dialysen.

Die bisher bekannten Anordnungen verwenden eine oder zwei Pumpen und zusätzlich eine oder mehrere Klemmen, wobei Ausführungen bekannt sind, bei denen der Dialysator zwischen zwei Pumpen angeordnet ist oder bei welchen er stromab der venösen Pumpe vorgesehen ist. Weiterhin gibt es Vorrichtungen mit zusätzlichen, teilweise mit Luft gefüllten Kammern, welche beispielsweise in Form eines Windkessels, oder einer Expansionskammer ausgebildet sein können, oder Beuteln, welche vor der arteriellen Pumpe, zwischen den Pumpen oder nach der venösen Pumpe vorgesehen sein können. Diese bekannten Vorrichtungen werden durch Steuerungen betätigt, wobei Steuerungen bekannt sind, die zeit-zeitabhängig, druck-druckabhängig, druck-zeitabhängig, volumen-volumenabhängig oder druck-volumenabhängig gesteuert werden.

Die Europäische Patentanmeldung EP-A-0 229 271 offenbart eine Dialysevorrichtung für die Einnadeldialyse, die in der Arterienleitung eine erste Pumpe enthält, die in der ersten Phase Blut vom Patienten in eine Expansionskammer fördert, während die Venenleitung abgesperrt ist. Gleichzeitig fördert eine zweite Pumpe Blut aus der Expansionskammer zu einem Dialysator. Diese Regelung erfolgt in Abhängigkeit von dem Druck in der Venenleitung, so daß der durch Ultrafiltration bewirkte Druckabfall ausgeglichen wird. In der zweiten Phase steht die erste Pumpe und sperrt die Arterienleitung ab, während die zweite Pumpe Blut aus der Expansionskammer mit konstanter Geschwindigkeit zum Dialysator fördert.

Bei sämtlichen bekannten Anordnungen wird eine einzige Kanüle oder ein einlumiger Katheder verwendet, welcher mit dem Patienten verbunden ist. Von dieser Kanüle bzw. diesem Katheder zweigt ein arterieller und ein venöser Teil des Schlauchsystems ab, wobei diese Zweige entweder durch eine okkludierende Pumpe oder eine Klemme druckmäßig vom Rest des Systems abgekoppelt werden können. Weiterhin ist allen Vorrichtungen gemeinsam, daß ein mehr oder weniger großer Anteil des bereits dialysierten Blutes erneut in den extrakorporalen Kreislauf gepumpt wird, bevor das dialysierte Blut in den Körper zurückgelangt. Diesen Vorgang bezeichnet man als Rezirkulation.

Die Rezirkulation des Blutes kann unterschiedliche Ursachen haben. Es ist zunächst auf die Verhältnisse in der Fistel zu verweisen. Je nachdem, ob der den Blutfluß bestimmende Strömungswiderstand stromauf, d.h. auf der arteriellen Seite oder stromab, d.h. auf der venösen Seite der Fistel liegt, ergeben sich unterschiedliche Strömungsverhältnisse. Liegt der Strömungswiderstand arteriell, so wird während der arteriellen Pumpphase mehr Blut aus der Fistel gepumpt, als arteriell angeliefert wird. Diese führt dazu, daß ein Teil des Blutes, das während der venösen Phase in den Teil der Fistel stromab der Kanüle gepumpt wird, zurückgesaugt und somit rezirkuliert wird. Analoge Verhältnisse liegen vor, falls stromab der Kanüle ein Strömungswiderstand vorliegt. In diesem Fall wird während der venösen Pumpphase ein Teil des Blutes in den Bereich stromauf der Kanüle gepumpt, das dann während der arteriellen Phase wieder angesaugt und somit rezirkuliert wird.

Hinsichtlich der Strömungsverhältnisse in der Kanüle ist darauf hinzuweisen, daß am Ende der venösen Phase die Kanüle vollständig mit gereinigtem Blut gefüllt wird. Dieses wird bei der nächsten arteriellen Phase wieder in den extrakorporalen Kreislauf gesaugt und somit rezirkuliert. Es wurde versucht, dieses Problem dadurch zu verringern, daß die jeweiligen Kanülen und Katheder kleine Füllvolumina aufweisen, wobei hierdurch das Problem der Rezirkulation nicht in zufriedenstellender Weise gelöst werden kann.

Ein weiterer Aspekt der Rezirkulation ergibt sich aus dem verwendeten Schlauchsystem. Während der venösen Pumpphase wird das gesamte Schlauchsystem, welches zwischen der venösen Pumpe und der arteriellen Pumpe oder Klemme liegt, dem venösen Rücklaufdruck ausgesetzt. Aufgrund der Nachgiebigkeit der Schläuche und des eventuell in der arteriellen Druckableitung vorhandenen Luftpolsters nimmt das Füllvolumen dieses Teils des extrakorporalen Schlauchsystems zu, das Schlauchsystem wird "aufgebläht". Am Ende der venösen Phase ist zwischen der venösen Klemme und der arteriellen Pumpe bzw. Klemme ein gegenüber dem drucklosen Zustand zusätzliches Volumen gereinigten Blutes im Schlauchsystem gespeichert. In der unmittelbar darauffolgenden arteriellen Phase wird dieses Volumen rezirkuliert. Da während dieser arteriellen Phase ein Unterdruck auftritt, sinkt das Füllvolumen gegenüber dem drucklosen Zustand, es wird also zusätzlich gereinigtes Blut aus dem venösen Schlauchstück angesaugt und somit rezirkuliert. Dieser Vorgang wiederholt sich bei jedem Zyklus. Es sind Vorschläge bekannt, dieses Problem durch zusätzliche Klemmen oder durch speziell ausgebildete Schlauchleitungen zu lösen. All diese Maßnahmen haben sich aus praktischen Gründen nicht durchsetzen können, da die zusätzlichen Klemmen direkt am Patienten angeordnet werden müssen, wodurch dieser in seiner Bewegungsfreiheit erheblich eingeschränkt wird. Die Verwendung spezieller, nicht dehnbarer Schlauchleitungen bringt sowohl in wirtschaftlicher als auch in technischer Hinsicht, beispielsweise hinsichtlich der Materialprobleme, erhebliche Nachteile.

Ein weiterer Faktor, welcher die Rezirkulation bestimmt, ergibt sich aus elektronischen oder mechanischen Verzögerungszeiten in dem arteriellen und dem venösen Steuerkreis. So kann es beispielsweise vorkommen, daß die arterielle Pumpe bereits anläuft, wenn die venöse Pumpe noch fördert bzw. die venöse Klemme noch geöffnet ist. Bei den bisher bekannten Vorrichtungen lassen sich diese Effekte nicht soweit beseitigen, daß hierdurch die Rezirkulation in wirksamer Weise unterbunden oder vermindert werden könnte.

Die Rezirkulation beeinflußt in entscheidendem Maße die Effektivität des extrakorporalen Kreislaufs und somit beispielsweise der Dialyse, da der durch den Dialysator strömende Volumenstrom um den Rezirkulationsanteil größer ist, als die Blutmenge, welche die Fistel tatsächlich verläßt.

Der Erfindung liegt die Aufgabe zugrunde, ein Verfahren und eine Vorrichtung der genannten Art zu schaffen, welche die negative Auswirkung der Rezirkulation auf die Effektivität weitgehend vermeidet und somit der Wirkungsgrad gesteigert wird und bei welchem die Vorrichtung bei einfachem Aufbau und sicherer Wirkungsweise so betätigbar ist, daß eine zeitlich von der Rezirkulation unabhängige Blutmenge von dem Patienten entnommen bzw. diesem rückgeführt werden kann.

Hinsichtlich des Verfahren wird die Erfindung dadurch gelöst, daß die arterielle und die venöse Pumpe, deren Pumpraten aufgrund von Sollwerten, die durch Regeleinrichtungen vorgegeben sind, eingestellt werden, zu Beginn der jeweiligen Pumpphase mit einer gegenüber einer vorgegebenen Rate erhöhten Förderrate betrieben werden, bis sich ein vorgegebener Druck eingestellt hat und dann über den Rest der jeweiligen Pumpphase bei der vorgegebenen Rate betrieben werden.

Das erfindungsgemäße Verfahren zeichnet sich durch eine Reihe erheblicher Vorteile aus. Das Verständnis der Erfindung wird erleichtert, wenn zunächst der zeitliche Ablauf der Rezirkulation näher betrachtet wird. Am Ende der venösen Pumpphase herrscht im Schlauchsystem der positive venöse Rücklaufdruck. Dabei wurde, bedingt durch die Dehnbarkeit des Schlauchsystems ein bestimmtes Volumen an bereits gereinigtem Blut in das arterielle Schlauchstück gedrückt. Es befindet sich dort im unmittelbar an der Kanüle angrenzenden Schlauchabschnitt.

Zu Beginn der arteriellen Phase, d.h. während des Übergangs vom positiven venösen Rücklaufdruck zum negativen arteriellen Ansaugdruck wird zunächst dieses im arteriellen Schlauch gespeicherte Blutvolumen und danach erst das im venösen Schlauch gespeicherte Blutvolumen und erst allmählich Blut aus dem Patienten gefördert. Es kommt somit zu einer Segmentierung des Blutes mit dem bereits gereinigten Blut an der Spitze des von der arteriellen Pumpe geförderten Flüssigkeitszylinders. Während dieser Anfangsphase wird kein Blut aus dem Patienten gefördert. Beim Übergang von der arteriellen zur venösen Phase erfolgt eine analoger Vorgang.

Das erfindungsgemäße Verfahren vermeidet die Nachteile dieser Vorgänge. Da erfindungsgemäß jeweils nach dem Einschalten der Pumpe ein maximaler Volumenstrom gefördert wird, erfolgt ein beschleunigtes Fördern des Speichervolumens, erst nach dem Abpumpen dieses Speichervolumens wird die jeweilige Pumpe auf die vorgegebene Rate zurückgeschaltet.

Erfindungsgemäß können die Pumpen beispielsweise in Abhängigkeit von einem vorgegebenen Pumpraten-Zeitprofil betätigt werden. Es ist jedoch erfindungsgemäß auch möglich, die Pumpen über eine Drucksteuerung zu betätigen. Zu diesem Zwecke wird die arterielle Pumpe so betrieben, daß sich nach dem Einschalten dieser Pumpe in kürzest möglicher Zeit ein vorgegebener arterieller Unterdruck einstellt und daß dieser während der ganzen Phase konstant gehalten wird.

Da erfindungsgemäß nach dem Umschalten zunächst der positive venöse Rücklaufdruck anliegt, wird die Druckregelung innerhalb der durch die Regelschaltung zwingend vorgegebenen Zeit die Pumpe solange mit einer maximalen Rate betreiben, bis der vorgegebene Druckwert erreicht ist, erst danach wird die Pumpe so betrieben, daß eine konstante Förderung erfolgt, ohne daß dabei eine Schädigung der Fistel auftritt. Durch diese Vorgehensweise wird erreicht, daß der effektive Blutfluß aus bzw. zum Patienten maximiert wird. Es ist somit erfindungsgemäß eine erhebliche Erhöhung des Wirkungsgrades gegeben.

Obenstehend wurde die Erfindung hinsichtlich der Betätigung der arteriellen Pumpe beschrieben, es versteht sich für den Fachmann, daß die venöse Pumpe in analoger Weise betätigt wird und daß sich hierbei analoge Effekte ergeben.

Bezüglich der Vorrichtung wird die erfindungsgemäße Aufgabe dadurch gelöst, daß der jeweils in dem arteriellen bzw. dem venösen Schenkel befindliche Drucksensor über eine insbesondere den Einschaltvorgang der jeweiligen Pumpe regelnde Regeleinrichtung mit der Steuereinheit verbunden ist und daß die Regeleinrichtung mit der jeweiligen zugeordneten Pumpe betriebsverbunden ist. Est ist erfindungsgemäß bei der Vorrichtung somit eine zusätzliche Regeleinrichtung vorgesehen, welche den Einschaltvorgang der Pumpe regelt, um diese mit einer maximalen Förderleistung zu Beginn des Einschaltvorganges zu betreiben und die Förderleistung dann zur Aufrechterhaltung eines konstanten Fördervolumens abzusenken.

Zum Verständnis der Erfindung ist besonders darauf hinzuweisen, daß die herkömmlichen Drucksteuerungen, welche bei Single-Needle-Anordnungen bekannt sind, sich lediglich auf die Druckverhältnisse während des Fördervorgangs selbst beziehen und somit zusätzlich zu der erfindungsgemäßen Steuerung vorgesehen sein können, da diese bekannten Steuerungsverfahren sich auf das Umschalten der Pumpphasen beziehen, während erfindungsgemäß die Pumprate, d.h. die Förderleistung der Pumpe geregelt wird.

Im folgenden wird die Erfindung anhand eines Ausführungsbeispiels in Verbindung mit der Zeichnung beschrieben. Dabei zeigt:
- Fig. 1: Pumpraten-Zeitprofile der arteriellen und der venösen Pumpe und
- Fig. 2: eine schematische Darstellung der erfindungsgemäßen Vorrichtung.

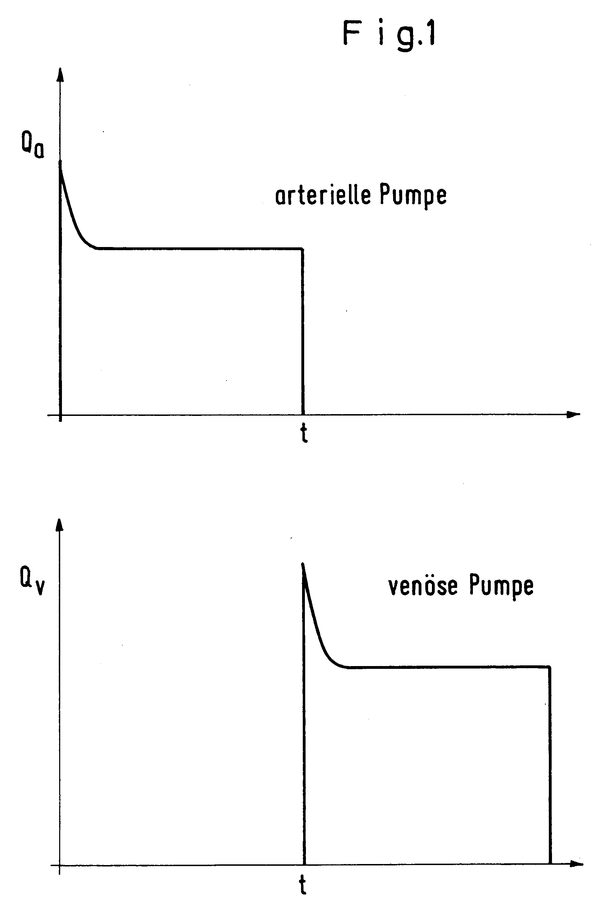

In Fig. 1 ist in Form eines Diagramms jeweils die Abhängigkeit der Pumpleistung von der Betätigungszeit der Pumpe dargestellt. Es ist dabei deutlich zu erkennen, daß beim Einschalten der Pumpe diese mit einer maximalen Förderrate betrieben wird und daß die Förderrate nachfolgend auf einen konstanten Wert abgesenkt wird, um dem Patienten eine konstante Blutmenge ab- bzw. zuzuführen.

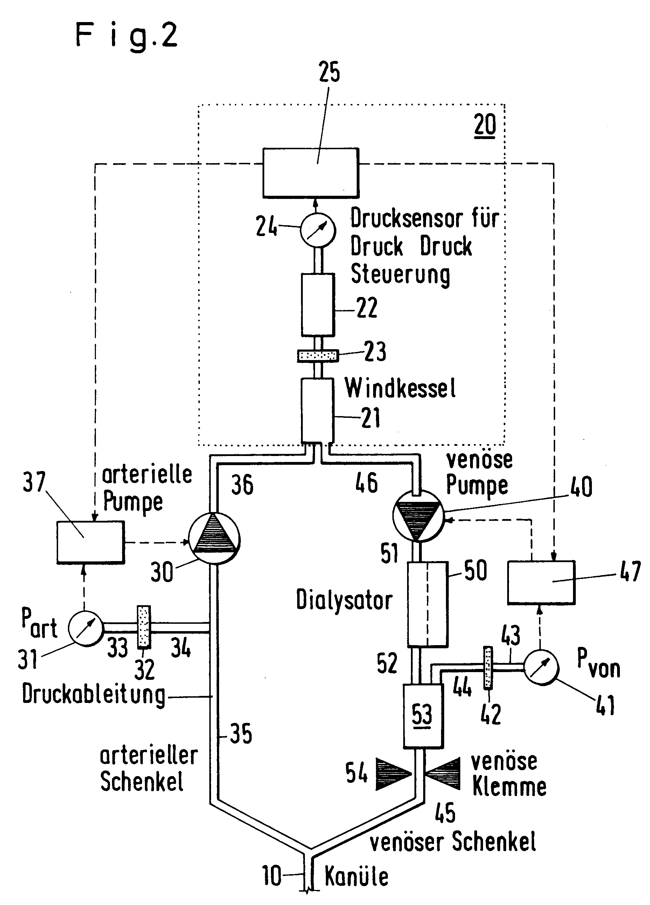

Die Fig. 2 zeigt eine schematische Darstellung der erfindungsgemäßen Anordnung für eine Single-Needle-Dialyse.

Die Vorrichtung weist eine Kanüle 10 auf, welche einlumig ausgeibldet ist und mit einem Patienten verbunden werden kann. Die Kanüle 10 verzweigt sich in einen arteriellen Schenkel 35 und einen venösen Schenkel 45. In dem arteriellen Schenkel 35 ist eine arterielle Pumpe 30 angeordnet, das stromab der arteriellen Pumpe 30 verlaufende Schlauchstück 36 ist mit einem Windkessel 21 verbunden. Das aus dem Windkessel 21 austretende Schlauchstück 46 ist mit einer venösen Pumpe 40 betriebsverbunden, welche in dem venösen Schenkel 45 vorgesehen ist. Stromab der venösen Pumpe 40 ist ein Schlauchstück 51 vorgesehen, welches mit einem Dialysator 50 verbunden ist. Aus dem Dialysator 50 wird das behandelte Blut über ein Schlauchstück 52 einem Blasenfänger 53 zugeführt. Stromab des Blasenfängers 53 ist eine venöse Klemme 54 angeordnet.

Der Windkessel 21 ist in üblicher Weise mit einem hydrophoben Filter 23 verbunden, welchem ein zweiter Windkessel 22 folgt, an dessen Ausgangsseite ein Drucksensor 24 vorgesehen ist. Die von dem Drucksensor 24 abgegebenen Druckwerte dienen der Druck-Drucksteuerung mittels einer Steuereinheit 25.

Die Steuereinheit 25 ist jeweils betriebsverbunden mit einer Regeleinrichtung 37, 47, welche wiederum der Betätigung der Pumpen 30 bzw. 40 dient.

Sowohl in dem arteriellen Schenkel als auch in dem venösen Schenkel ist jeweils ein Drucksensor 31, 41 vorgesehen, mittels dessen der Ansaugdruck der arteriellen Pumpe 30 in dem arteriellen Schenkel 35 bzw. der Förderdruck der venösen Pumpe 40 in dem venösen Schenkel 45 gemessen werden kann. Der Drucksensor 31 ist mit dem arteriellen Schenkel 35 über Schlauchstücke 33, 34 und einen zwischengeschalteten Hydrophobfilter 32 verbunden. In analoger Weise erfolgt ein Anschluß des Drucksensors 41 über Schlauchstücke 43, 44, zwischen denen ein Hydrophobfilter 42 angeordnet ist.

Die Ausgangswerte der beiden Drucksensoren 31, 41, werden jeweils den Regeleinrichtungen 37, 47 zugeführt.

Anstelle des Dialysators 50 kann auch eine andere Blutbehandlungseinrichtung vorgesehen sein, z.B. ein Plasmafilter oder eine Absorptionseinrichtung.

Abgesehen von den Regeleinrichtungen 37 und 47 und der entsprechenden Betriebsverbindung mit den zu regelnden Bauelementen bzw. der Steuereinheit 25 entspricht die gezeigte Anordnung im wesentlichen dem Stand der Technik. Auf eine detaillierte Funktionsbeschreibung kann somit verzichtet werden.

Die beiden Regeleinrichtungen 37 und 47 bewirken, daß beim Einschalten sowohl die arterielle als auch die venöse Pumpe durch die Steuereinheit 25 auf einen vorgegebenen Ansaugdruck bzw. Förderdruck geregelt wird, wobei typische Werte zwischen - 100 mmHg und - 200 mmHg für die arterielle Pumpe und + 200 mmHg bis + 300 mmHg für die venöse Pumpe liegen.

Die Erfindung ist nicht auf das gezeigte Ausführungsbeispiel beschränkt, es ergeben sich vielmehr für den Fachmann im Rahmen der Erfindung vielfältige Abwandlungs- und Modifikationsmöglichkeiten.

## Patentansprüche

1. Verfahren zur Steuerung von Blutpumpen bei einem extrakorporalen Kreislauf mit einer Single-Needle-Anordnung mit einer mit dem Patienten verbundenen Kanüle (10) und jeweils einem von der Kanüle (10) abzweigenden arteriellen (35) und venösen (46) Schenkel, in welchen jeweils eine arterielle (30) bzw. eine venöse (40) Pumpe angeordnet sind, dadurch gekennzeichnet, daß die arterielle (30) und venöse (40) Pumpe, deren Pumpraten aufgrund von Sollwerten, die durch Regeleinrichtungen (37, 47) vorgegeben sind, eingestellt werden, zu Beginn der jeweiligen Pumpphase mit einer gegenüber einer vorgegebenen Rate erhöhten Förderrate betrieben werden, bis sich ein vorgegebener Druck eingestellt hat und dann über den Rest der jeweiligen Pumpphase bei der vorgegebenen Rate betrieben werden.

2. Verfahren nach Anspruch 1, dadurch gekennzeichnet, daß mittels eines in Strömungsrichtung vor der arteriellen Pumpe (30) angeordneten Druckaufnehmers (31) eine Drucksteuerung der arteriellen Pumpe (30) so erfolgt, daß sich nach dem Einschalten der Pumpe (30) in kürzest möglicher Zeit ein vorgegebener Unterdruck einstellt.

3. Verfahren nach Anspruch 2, dadurch gekennzeichnet, daß der Unterdruck während der gesamten Phase konstant aufrechterhalten wird.

4. Verfahren nach Anspruch 2 oder 3, dadurch gekennzeichnet, daß die arterielle Pumpe (30) bis zum Erreichen des vorgegebenen Unterdrucks mit maximaler Förderleistung und damit mit einer zur Aufrechterhaltung des Unterdrucks ausreichenden Förderleistung betrieben wird.

5. Verfahren nach einem der Ansprüche 1 - 4, dadurch gekennzeichnet, daß mittels eines stromab der venösen Pumpe (40) angeordneten Druckaufnehmers (41) eine Drucksteuerung der venösen Pumpe (40) so erfolgt, daß sich nach dem Einschalten der Pumpe (40) in kürzest möglicher Zeit ein vorgegebener Überdruck einstellt.

6. Verfahren nach Anspruch 5, dadurch gekennzeichnet, daß der Überdruck während der gesamten Phase konstant aufrechterhalten wird.

7. Verfahren nach Anspruch 5 oder 6, dadurch gekennzeichnet, daß die venöse Pumpe (40) bis zum Erreichen des vorgegebenen Überdrucks mit maximaler Förderleistung und damit mit einer zur Aufrechterhaltung des Überdrucks ausreichenden Förderleistung betrieben wird.

8. Verfahren nach einem der Ansprüche 1 - 7, dadurch gekennzeichnet, daß die arterielle Pumpe (30) beim Einschalten auf einen Ausangdruck von -100 mmHg bis -200 mmHg geregelt wird.

9. Verfahren nach einem der Ansprüche 1 - 8, dadurch gekennzeichnet, daß die venöse Pumpe (40) beim Einschalten auf einen Förderdruck von + 200 mmHg bis + 300 mmHg geregelt wird.

10. Vorrichtung zur Durchführung des Verfahrens nach einem der Ansprüche 1 - 9, mit einer einzigen, einlumigen, mit dem Patienten zu verbindenden Kanüle (10), welche in einen arteriellen (35) und einen venösen (45) Schenkel übergeht, mit einer arteriellen (30) und einer venösen (40) Pumpe, einem Blutbehandlungsgerät (50), einer Steuereinheit (25) und einem, mit der Steuereinheit (25) verbundenen Drucksensor (41) in dem venösen (45) Schenkel, dadurch gekennzeichnet, daß auch der arterielle Schenkel (35) einen mit der Steuereinheit (25) verbundenen Drucksensor (31) aufweist, daß die Drucksensoren (31, 41) jeweils über eine insbesondere den Einschaltvorgang der jeweiligen Pumpe (30, 40) regelnde Regeleinrichtung (37, 47) mit der Steuereinheit (25) verbunden ist und daß die Regeleinheit (37, 47) mit der jeweils zugeordneten Pumpe (30, 40) betriebsverbunden ist.

11. Vorrichtung nach Anspruch 10, dadurch gekennzeichnet, daß im Bereich zwischen der arteriellen (30) und der venösen (40) Pumpe ein mit der Steuereinheit (25) verbundener Drucksensor (24) für die druckabhängige Steuerung der Pumpen (30, 40) angeordnet ist.

## Claims

1. Process for the control of blood pumps in an extra-corporeal circuit having a single needle arrangement comprising with a canula (10) connected to the patient and, respectively branching from said canula (10), an arterial branch (35) and a venous branch (46), comprising respectively an arterial pump (30) and a venous pump (40), characterized in that the arterial pump (30) and the venous pump (40), upon activation, at a pumping rate higher than the normal throughput rate of the appropriate pumping phase.

2. Process according to claim 1, characterized in that by means of a pressure sensor (31) uptake means in the flow direction before the arterial pump (30) a pressure control of the arterial pump (30) is effected to obtain predetermined under-pressure in the shortest possible time after the activation of the pump (30).

3. Process according to claim 2, characterized in that the predetermined under-pressure is maintained at a constant level during the entire phase.

4. Process according to claim 2 or 3, characterized in that the arterial pump (30) is driven at its maximum throughput until the achievement of the desired under-pressure and thus with a sufficient throughput to maintain said under-pressure.

5. Process according to one of claims 1-4, characterized in that by means of a pressure sensor (41) upstream of the venous pump (40) a pressure control of the venous pump (40) is effected to obtain a predetermined high-pressure in the shortest possible time after the activation of the pump (40).

6. Process according to claim 3, characterized in that the high-pressure is maintained at a constant level during the entire phase.

7. Process according to claim 5 or 6, characterized in that the venous pump (40) is driven at its maximum thoughput until the achievement of the desired back-pressure and thus with a sufficient througput to maintain said high-pressure.

8. Process according to one claims of 1-7, characterized in that upon activation, the arterial pump (30) is controlled to a suction pressure of -100 mmHg to -200 mmHg.

9. Process according to one of claims 1-8, characterized in that upon activation, the venous pump (40) is controlled to a delivery pressure of +200 mmHg to +300 mmHg.

10. Device for conducting the process according to one of caims 1-9, having a sole monolumenar canula (10) connectible to the patient passing into an arterial branch (35) and to a venous branch (45) with an arterial (30) and a venous (40) pump, a blood treating device (50), a control unit (25) and a pressure sensor (41), connected to said control unit (25) in said venous (45) branch, characterized in that also said arterial branch (35) comprises a pressure sensor (31) connected to said control unit (25), that said pressure sensors (31, 41) are respectively connected to said control unit (25) via a regulating arrangement (47), particularly regulating the switching-on process of the respective pump (30, 40) and that said regulating arrangement (37, 47) is in operating connection with the respectively associated pump (30, 40).

11. Device according to claim 10, characterized in that in the area between the arterial (30) and the venous (40) pump a pressure sensor (24) connected to the control unit (25) is provided for the pressure-dependent control of said pumps (30,40).

## Revendications

1. Procédé pour commander des pompes à sang dans un circuit extracorporel, comportant un dispositif Single-Needle, comprenant une canule (10) reliée au patient et respectivement une branche artérielle (35) et une branche veineuse (46), qui s'étendent en dérivation à partir de la canule (10) et dans lesquelles sont disposées respectivement une pompe artérielle (30) et une pompe veineuse (40), caractérisé en ce que la pompe artérielle (30) et la pompe veineuse (40), dont les débits sont réglés sur la base de valeurs de consigne, qui sont prédéterminées par des dispositifs de régulation (37, 47), sont entraînés au début de la phase respective de pompage avec un débit de refoulement accru par rapport à un débit prédéterminé, jusqu'à ce qu'une pression prédéterminée se soit établie, puis sont entraînés avec le débit prédéterminé, pendant le reste de la phase respective de pompage.

2. Procédé selon la revendication 1, caractérisé en ce qu'une commande de la pression de la pompe artérielle (30) est réalisée au moyen d'un capteur de pression (31), qui est disposé dans la direction d'écoulement en amont de la pompe artérielle (30) de sorte qu'après l'activation de la pompe (30), une dépression prédéterminée est réglée pendant l'intervalle de temps le plus bref possible.

3. Procédé selon la revendication 2, caractérisé en ce que la dépression est maintenue constante pendant l'ensemble de la phase.

4. Procédé selon la revendication 2 ou 3, caractérisé en ce que la pompe artérielle (30) est entraînée jusqu'à l'obtention de la dépression prédéterminée avec la puissance d'entraînement maximale et par conséquent, avec une puissance de refoulement qui suffit pour le maintien de la dépression.

5. Procédé selon l'une des revendications 1-4, caractérisé en ce qu'une commande de pression de la pompe veineuse (40) est réalisée au moyen d'un capteur de pression (41) disposé en aval de la pompe veineuse (40) de telle sorte qu'après l'activation de la pompe (40), une surpression prédéterminée est réglée pendant un intervalle de temps aussi court que possible.

6. Procédé selon la revendication 5, caractérisé en ce que la surpression est maintenue constante pendant toute la phase.

7. Procédé selon la revendication 5 ou 6, caractérisé en ce que la pompe veineuse (40) fonctionne jusqu'à ce que la surpression prédéterminée soit atteinte, avec une puissance de refoulement maximale et par conséquent avec une puissance de refoulement qui suffit pour le maintien de la surpression.

8. Procédé selon l'une des revendications 1-7, caractérisé en ce que la pompe artérielle (30) est réglée sur une pression d'aspiration comprise entre -100 mmHg et - 200 mmHg, lors de son activation.

9. Procédé selon l'une des revendications 1-8, caractérisé en ce que la pompe veineuse (40) est réglée sur une pression de refoulement comprise entre +200 mmHg et +300 mmHg lors de son activation.

10. Dispositif pour la mise en oeuvre du procédé selon l'une des revendications 1 à 9, comportant une seule canule à un seul passage (10), destinée à être raccordée au patient et qui se prolonge par une branche artérielle (35) et une branche veineuse (45), et comportant une pompe artérielle (30) et une pompe veineuse (40), un appareil (50) de traitement du sang, une unité de commande (25) et un capteur de pression (40), qui est relié à l'unité de commande (25) et est situé dans la branche veineuse (45), caractérisé en ce que, également, la branche artérielle (35) possède un capteur de pression (31) raccordé à l'unité de commande (25), que les capteurs de pression (21, 31) sont reliés à l'unité de commande (25) respectivement par l'intermédiaire d'un dispositif de régulation (37, 47), qui règle notamment l'opération d'activation de la pompe respective (30, 40), et que l'unité de réglage (37, 47) est raccordée en fonctionnement à la pompe respectivement associée (30, 40).

11. Dispositif selon la revendication 10, caractérisé en ce qu'un capteur de pression (24), relié à l'unité de commande (25) et servant à commander les pompes (30, 40) en fonction de la pression, est disposé dans la zone située entre la pompe artérielle (30) et la pompe veineuse (40).
